(19) Europäisches Patentamt
European Patent Office
Office européen des brevets

(11) Numéro de publication: **0 037 388**
**B1**

(12) **FASCICULE DE BREVET EUROPEEN**

(45) Date de publication du fascicule du brevet: **12.12.84**

(51) Int. Cl.$^3$: **A 61 K 47/00,** A 61 K 9/18

(21) Numéro de dépôt: **81870017.1**

(22) Date de dépôt: **30.03.81**

# (54) Formes pharmaceutiques, leur préparation et les compositions qui les contiennent.

(30) Priorité: **31.03.80 BE 200041**

(43) Date de publication de la demande:
**07.10.81 Bulletin 81/40**

(45) Mention de la délivrance du brevet:
**12.12.84 Bulletin 84/50**

(84) Etats contractants désignés:
**BE CH DE FR GB IT LI NL**

(56) Documents cités:
**WO-A-79/00515**
**BE-A- 882 541**
**GB-A-1 541 436**
**US-A-3 167 485**
**US-A-4 017 471**

**UNLISTED DRUGS, vol. 29, no. 5, mai 1977 CHATHAM (US) page 74K**

(73) Titulaire: **INSTITUT INTERNATIONAL DE PATHOLOGIE CELLULAIRE ET MOLECULAIRE**
**75 Avenue Hippocrate**
**B-1200 Bruxelles (BE)**

(72) Inventeur: **Trouet, André Benoit Léon**
**29, Predikherenberg**
**B-3009 Winksele (BE)**
Inventeur: **Masquelier, Michèle Mariette L.G.**
**126, Rue Charles Degroux**
**B-1040 Bruxelles (BE)**
Inventeur: **Baurain, Roger Marcel**
**72, Avenue des Violettes**
**B-1970 Wezembeek-Oppem (BE)**

(74) Mandataire: **de Kemmeter, François et al**
***Cabinet Bede* 13, *avenue Antoine Depage***
**B-1050 Bruxelles (BE)**

**EP 0 037 388 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne de nouvelles formes pharmaceutiques d'un principe actif, leur préparation et les compositions qui les contiennent.

Un médicament ou une drogue, pour exercer son effet, doit pouvoir pénétrer dans la cellule appropriée appelée cellule cible. Généralement cette pénétration s'effectue par diffusion du médicament ou de la drogue à travers la paroi cellulaire. Dans ces conditions, il s'établit un équilibre entre la concentration du principe actif dans la cellule et la concentration dans le milieu extérieur. Par ailleurs, le médicament ne pénètre pas toujours sélectivement dans les cellules cibles sur lesquelles il doit agir et, par son action sur les cellules saines, le médicament peut exercer des effets secondaires néfastes qui peuvent nuire à son utilité.

Un autre modèle de pénétration intracellulaire est basé sur un mécanisme endocytaire au cours duquel les macromolécules sont incluses dans des vacuoles formées par l'invagination de la membrane cellulaire avant de fusionner avec les lysosomes.

Ce mécanisme endocytaire dont la prépondérance dépend du type cellulaire peut être utilisé en thérapeutique pour augmenter la sélectivité des agents thérapeutiques vis-à-vis des cellules susceptibles de ce mécanisme.

Pour que l'agent thérapeutique ait une taille suffisante pour participer à ce mécanisme endocytaire, il est possible de lier le principe actif à une macromolécule, telle qu'une protéine, qui sera désignée sous le nom de vecteur.

Des combinaisons agent thérapeutique-vecteur sont déjà connues.

Ainsi, le brevet américain No 4.017.471 divulgue la liaison d'agents antitumoraux à des anticorps spécifiques pour certaines tumeurs par des liens peptidiques. Aucun bras intermédiaire n'est prévu entre l'agent actif et le verteur anticorps.

La demande internationale publiée WO—A—79/00515 prévoit la liaison d'un médicament à un polymère vecteur; par exemple un polymère ou copolymère d'aminoacide par liaison covalente, c'est-à-dire notamment par l'intermédiaire de "molécules bras" (spacer molecules) dont on donne comme exemples des oligopeptides, l'anhydride succinique ou l'anhydride maléique.

La présente invention part des constatations suivantes.

Pour que le médicament puisse exercer en totalité ou en partie ses effets, il est nécessaire qu'il soit libéré à l'intérieur ou au voisinage immédiat de la cellule cible. En d'autres termes, la combinaison principe actif-vecteur ou drogue-vecteur doit répondre aux trois critères suivants:

1) être stable dans la circulation sanguine avant d'atteindre les cellules cibles,
2) être dissociée ou scindée dans les cellules cibles ou dans leur voisinage immédiat,
3) être capable de régénérer la drogue sous sa forme active.

Pour que le premier de ces critères soit satisfait, il est nécessaire que le lien entre la drogue et le vecteur soit de nature covalente, et que ce lien covalent résiste aux hydrolases présentes dans le sérum.

La combinaison drogue-vecteur pénétrant dans les cellules cibles par un mécanisme essentiellement endocytaire, il est nécessaire de tenir compte des propriétés des lysosomes pour satisfaire au second critère. En effet, le lien entre la drogue et le vecteur peut être coupé soit chimiquement en milieu acide soit enzymatiquement au moyen des hydrolases acides des lysosomes. Mais encore faut-il, compte tenu du troisième critère, que la drogue soit libérée sous sa forme active. Il a été constaté qu'un tel résultat ne peut pas être obtenu si le principe actif est lié directement à la macromolécule.

Il a été trouvé, et c'est ce qui fait l'objet de la présente invention, qu'on peut obtenir un médicament qui possède les caractéristiques désirées, si on intercale un lien temporaire désigné par le terme "bras", entre la drogue et le vecteur.

La présente invention concerne les nouvelles formes pharmaceutiques d'un principe actif qui peuvent être schématiquement représentées par:

Vecteur—Bras—Drogue (I)

leur procédé de préparation et les compositions qui les contiennent.

Selon l'invention, la "drogue" est constituée par un principe actif qui agit intracellulairement et qui contient dans sa molécule une fonction amine libre. Les drogues à fonction amine libre faisant l'objet de la présente demande comprennent notamment des anthracyclines ayant une activité antitumorale, telles que la daunorubicine ou la doxorubicine, ou des quinoléines ayant des propriétés antimalariques et antileishmaniales, telles que la primaquine.

Selon l'invention, le "bras" est de nature peptidique et il contient, de préférence, 3 ou 4 aminoacides. Il est particulièrement important que l'aminoacide, lié à la fonction amine libre de la "drogue" par sa fonction carboxylique, comporte un carbone asymétrique et possède la configuration L car les peptidases ou les hydrolases lysosomiales ne coupent que les liaisons peptidiques ne $\alpha$ d'un carbone asymétrique.

D'un intérêt tout particulier pour remplir cette fonction est la L-leucine.

Selon l'invention, le "bras" intermédiaire est représenté par l'enchaînement:

X—L—Leu

dans lequel L-leu est lié par sa fonction carboxylique à la fonction amine de la drogue et X représente 1, 2 ou 3 aminoacides, identiques ou différents, choisis parmi la L-alanine, la glycine et la L-leucine.

D'un intérêt tout particulier sont les "bras" peptidiques L-ala-L-leu, L-leu-L-ala-L-leu, L-ala-L-leu-L-ala-L-leu, $(L\text{-ala})_3$-L-leu, L-ala-L-leu-gly-L-leu, $(gly\text{-L-leu})_2$; parmi ceux-ci les enchainements dans lesquels la L-ala et la L-leu alternent conviennent particulièrement bien.

Par exemple, parmi les dérivés de la fonction amine des anthracyclines antitumorales, telles que la daunorubicine il a été montré que la N-L-leucyl-daunorubicine est le produit qui, dans les conditions de la digestion lysosomiale libère la quantité la plus importante de daunorubicine. Ainsi après 2 heures d'hydrolyse à pH 4,5, la N-L leucyl-daunorubicine libère 65% de daunorubicine libre et 90% à pH 6,0. Tandis que par exemple la L-tyrosine et la L-isoleucine, lorsqu'elles sont liées à la daunorubicine, régénèrent à pH 6,0 respectivement 70% et 60% de daunorubicine par l'action des peptidases lysosomiales. Cependant la L-leucine a été sélectionnée comme acide aminé adjacent à la drogue ou au principe actif car la N-L-leucyl-daunorubicine, la N-L-leucyl-doxorubicine et la N-L-leucyl-primaquine sont stables dans le sérum et régénèrent rapidement la drogue ou le principe actif quand elles sont soumis es à la digestion lysosomiale.

Si le bras entre la daunorubicine et le vecteur est une L-leucine, après 24 heures d'hydrolyse à pH 6,0 du produit vecteur-brase-drogue par des enzymes lysosomiaux purifiés, 15% de la drogue va être libérés sous forme de daunorubicine libre. De plus, un taux de libération de la "drogue" libre beaucoup plus important est obtenu lorsque le nombre des aminoacides constituant le "bras" peptidique augmente et il peut atteindre 78% lorsque le peptide intermédiaire est constitué par l'enchaînement de 4 aminoacides.

Selon l'invention, le "vecteur" est une macromolécule qui peut être endocytée sélectivement par les cellules cibles. De préference la macromolécule est une protéine dont la nature peptidique est liée à l'action recherchée de la "drogue". Par exemple, dans le cas des dérivés des anthracyclines antitumorales, le sérum albumine bovine (BSA), la fétuine, l'immunoglobuline ou les hormones peptidiques telles que la lactotropine ou l'hormone placentaire lactogène peuvent être avantageusement utilisées. Dans le cas des quinoléines antimalariques, il est particulièrement intéressant d'utiliser l'asialofétuine ou tout autre glycoprotéine porteuse de résidus galactosyles terminaux, qui vont être captées spécifiquement par les hépatocytes où se trouvent les parasites.

Dans le cas des quinoléines antileishmaniales, il est particulièrement intéressant d'utiliser des glycoprotéines porteuses de résidus mannosyles terminaux, qui vont être captées spécifiquement par les macrophages où résident les parasites.

Selon l'invention, les nouvelles formes pharmaceutiques peuvent être obtenues par action d'un produit de formule générale:

X—L—leu—Drogue (II)

dans laquelle le terme "Drogue" et le symbole X sont définis comme précédemment, sur une protéine dans les conditions habituellement utilisées pour créer une liaison peptidique entre la fonction amine libre de l'aminoacide terminal du produit de formule générale (II) et les fonctions acides libres de la protéine.

Généralement, la réaction s'effectue dans un milieu tamponné convenable tel qu'un milieu au tampon phosphate (Phosphate Buffered Saline ou PBS) en présence d'un agent de condensation tel qu'un carbodiimide comme l'éthyl -1 (diméthylamino-3 propyl)-3 carbodiimide à une température comprise entre 0 et 30°C. Il est particulièrement avantageux d'opérer en l'absence de lumière.

La nouvelle forme pharmaceutique selon l'invention est séparée du mélange réactionnel selon les méthodes physiques ou physicochimiques telles que la chromatographie sur un support approprié.

Le "bras" peut également être accroché aux fonctions amines libres de la protéine après succinylation de la fonction amine libre de l'aminoacide terminal du produit de formule générale (II) et activation de la fonction carboxylique terminale du succinyl par du N-hydroxysuccinimide. La condensation du bras porteur de la drogue avec le vecteur s'effectue alors en absence d'un agent de condensation, pour donner les nouvelles formes pharmaceutiques de formule générale (I).

Selon l'invention les nouvelles formes pharmaceutiques de formule générale (I) dans lesquelles le "bras" peptidique est lié au "vecteur" par l'intermédiaire d'un reste succinyle peuvent aussi être obtenues par action d'un produit de formule générale (II) sur la protéine succinylée.

Il est particulièrement avantageux d'opérer en présence d'un agent de condensation tel qu'un carbodiimide comme l'éthyl-1(diméthylamino-3 propyl)-3 carbodiimide à une température comprise entre 0 et 30°C et de préférence en l'absence de lumière.

Le produit de formule générale (II) peut être obtenu par condensation d'un aminoacide convenable ou d'un peptide sous forme acide dont les fonctions amines sont protégées, avec une drogue

portant une fonction amine libre ou un dérivé de cette drogue portant une fonction amine libre en présence d'un agent de condensation selon les méthodes habituelles utilisées en chimie peptidique.

De préférence, on réalise d'abord la condensation de la L-leucine dont la fonction amine est protégée, sur la "drogue" puis la condensation successive des aminoacides destinés à constituer le bras sur la N-L-leucyl-"Drogue".

Comme agent de condensation, on utilise plus particulièrement un carbodiimide dans un solvant organique tel que le diméthyl-formamide, le chlorure de méthylène ou l'acétonitrile. La fonction acide de l'aminoacide peut aussi être activée sous forme d'ester avec le N-hydroxysuccinimide ou sous forme d'anhydride mixte.

Les groupements protecteurs des fonctions amines sont des groupements de type acyle (formyle) ou uréthane (fluorénylméthoxycarbonyle, benzyloxycarbonyle, tertio-butyloxycarbonyle) ou arylalcoyle (trityle). Ces groupements peuvent ensuite être éliminés dans des conditions qui ne doivent pas détruire la liaison peptidique formée. Par exemple, lorsque la fonction amine est protégée par un radical trityle, ce groupement protecteur peut être éliminé par hydrolyse en milieu acide.

Les nouvelles formes pharmaceutiques selon l'invention sont particulièrement utiles pour permettre une action sélective dans le domaine d'activité de la drogue de base. L'action des nouveaux médicaments étant plus spécifique, le même effet sera obtenu avec une quantité plus faible de drogue; par ailleurs, les effets secondaires néfastes pourront être fortement atténués sinon supprimés grâce à la moindre dissémination de la drogue en dehors des cellules cibles.

Les nouveaux produits selon l'invention présentent des propriétés particulièrement intéressantes.

Ainsi les produits pour lesquels la drogue est la daunorubicine inhibent, in vitro, la croissance des cellules cancéreuses, telles que les cellules de la leucémie L 1210 dans un milieu approprié. Par exemple, après 72 heures de culture, la BSA-succinyl-L-Ala-L-Leu-L-Ala-L-Leu-Daunorubicine, à une concentration voisine de 10 $\mu g/cm^3$ provoque 90% d'inhibition de la croissance des cellules de leucémie L 1210.

Chez la souris, greffée par la leucémie L 1210 par voie intrapéritonéale et traitée par voie intrapéritonéale, la fétuine-succinyl-L-Ala-L-Leu-L-Ala-L-Leu-daunorubicine et la BSA-succinyl-L-Ala-L-Leu-L-Ala-L-Leu-daunorubicine manifestent, à doses égales exprimées en daunorubicine, une activité nettement supérieure à celle de la daunorubicine ou de la L-leucyl-daunorubicine, associée à une toxicité moindre.

Par exemple, à la dose de 5 mg/kg exprimée en daunorubicine, la fétuine-L-Ala-L-Leu-L-Ala-L-Leu-daunorubicine produit une augmentation du temps de survie (ILS) voisine de 194% avec 8 souris survivantes sur 10 après 30 jours d'observation, alors que, dans les mêmes conditions, la daunorubicine, déjà toxique à cette dose, provoque une augmentation du temps de survie (ILS) voisine de 7%.

Les produits selon l'invention pour lesquels la drogue est la primaquine provoquent, lorsqu'ils sont administrés par voie intra-veineuse à des doses comprises entre 10 et 30 mg/kg avant l'infestation par Plasmodium berghei une augmentation du temps de survie supérieure à 400% par rapport aux témoins non traités.

Les exemples suivants, donnés à titre non limitatif, montrent comment l'invention peut être mise en pratique.

Exemple 1

a) Préparation de la L-alanyl-L-leucyl-L-alanyl-L-leucyl-daunorubicine

A une solution glacée de 1 g de daunorubicine (1,77 mmoles) dans 200 $cm^3$ de tampon borate à pH 10,2, agitée sous azote, on ajoute une solution de 320 mg de N-carboxyanhydride de L-leucine (2,04 mmoles) dans 10 $cm^3$ d'acétone à $-10°C$. Après 5 minutes d'agitation, le pH est amené à 3,5 par addition d'acide sulfurique 6 N puis, après 15 minutes, le mélange réactionnel est neutralisé par addition de soude 1 N. La solution est extraite par 150 $cm^3$ de chlorure de méthylène. La phase organique est séchée sur sulfate de sodium. Après filtration et concentration à sec sous pression réduite (2666,4 Pa) à une température inférieure 50°C, le produit est purifiée par chromatographie sur 60 g de silice en éluant avec un mélange chloroforme-méthanol (95—5 en volumes). On obtient ainsi, après évaporation du solvant, 690 mg de N-L-leucyl-daunorubicine qui, par addition de la quantité théorique d'acide chlorhydrique, fournit 715 mg de chlorhydrate de L-leucyl-daunorubicine fondant à 201°C avec décomposition.

A une solution agitée de 677 mg de L-leucyl-daunorubicine (1 mmole) dans 16 $cm^3$ de diméthylformamide, on ajoute 498 mg de N-trityl-L-alaninate de N-hydroxysuccinimide (2,12 mmoles) et 0,140 $cm^3$ de triéthylamine. Le mélange réactionnel est agité pendant 24 heures à une température voisine de 20°C. Après évaporation du solvant, le résidu est dissous dans 10 $cm^3$ d'un mélange chloroforme-méthanol (99—1 en volumes), puis filtré sur une colonne de 45 g de gel silice. On obtient, après évaporation du solvant, 450 mg de N-trityl-L-alanyl-L-leucyl-daunorubicine. Ce produit est traité à froid par une solution d'acide acétique à 75%, puis la solution est neutralisée par addition d'ammoniaque 12 N.

Après extraction par le chloroforme, séchage sur sulfate de sodium, filtration et évaporation du solvant, formation du chlorhydrate et filtration du triphénylcarbinol, on obtient 470 mg de chlorhydrate de L-alanyl-L-laucyl-daunorubicine fondant à 205°C avec décomposition.

On dissout 250 mg de chlorhydrate de L-alanyl-L-leucyl-daunorubicine dans 6 $cm^3$ de diméthylformamide puis on ajoute 0,046 $cm^3$ de triéthylamine et 166 mg de N-trityl-L-leucinate de N-hydroxysuccinimide. En opérant comme précédemment pour la préparation de la L-alanyl-L-leucyl-daunorubicine, on obtient 130 mg de chlorhydrate de L-leucyl-L-alanyl-L-leucyl-daunorubicine fondant à 170°C avec décomposition.

On dissout 98 mg de chlorhydrate de L-leucyl-L-alanyl-L-leucyl-daunorubicine dans 5 $cm^3$ de diméthylformamide. On ajoute 0,016 $cm^3$ de triéthylamine et 57 mg de N-trityl-L-alaninate de N-hydroxysuccinimide. En opérant comme précédemment pour la préparation de la L-alanyl-L-leucyl-daunorubicine, on obtient 65,5 mg de chlorhydrate de L-alanyl-L-leucyl-L-alanyl-L-leucyl-daunorubicine fondant à 217°C avec décomposition.

b) Couplage avec la sérum albumine bovine.

A 8 mg de chlorhydrate de L-alanyl-L-leucyl-L-alanyl-L-leucyl-daunorubicine en solution dans 2 $cm^3$ d'eau distillée, on ajoute 100 mg de sérum albumine bovine et 50 mg de 1-ethyl-3-(3 diméthylaminopropyl)carbodiimide (Sigma Chem. USA) en solution dans 3 $cm^3$ de tampon phosphate (Phosphate Buffered Saline, PBS).

Le mélange est conservé pendant 15 heures à l'obscurité, à une température voisine de 20°C, puis la solution est filtrée sur une colonne de 150 $cm^3$ de Biogel P-100® en éluant avec du tampon phosphate (Phosphate Buffered Saline, PBS).

La première fraction (14 $cm^3$) qui correspond au produit couplé, est filtrée sur une colonne contenant 1 g de Porapak Q® (Waters) afin d'éliminer les traces éventuelles de drogue fixée de façon non covalente. La solution est stérilisée par filtration sur filtre Millipore® 0,2 $\mu$m et elle est conservée à −20°C.

On obtient ainsi une solution contenant 50 $\mu$g de drogue par $cm^3$ et 3 mg de sérum albumine bovine par $cm^3$.

Exemple 2

a) Succinylation de la N-L-alanyl-L-leucyl-L-alanyl-L-leucyl-daunorubicine

On dissout 130 mg de chlorhydrate de N-L-alanyl-L-leucyl-L-alanyl-L-leucyl-daunorubicine (0,139 mmoles) dans 15 $cm^3$ d'eau bidistillée.

On ajoute 2 fois 14 mg d'anhydride succinique (2 fois 0,14 mmoles) par petites fractions, tout en maintenant le pH à 7,5 par addition de soude 1 N.

A la fin de la réaction, le pH du mélange réactionnel est ajusté à 5,5 puis on extrait le produit formé par 3 fois 20 $cm^3$ de chloroforme. La phase organique est lavée soigneusement 3 fois à l'eau bidistillée, puis séchée sur sulfate de sodium.

Après évaporation de la phase organique, on obtient 100 mg de poudre rouge (rendement 72%) qui est mise en suspension dans 20 $cm^3$ d'eau. On ajoute 1 $cm^3$ d'une solution de soude 0,1 N. La solution ainsi obtenue est lyophilisée. On obtient ainsi 103 mg de sel de sodium de le succinyl-L-ala-L-leu-L-ala-L-leu-daunorubicine.

b) Couplage avec la sérum albumine bovine

On dissout 8,7 mg de succinyl-alanyl-leucyl-alanyl-leucyl-daunorubicine dans 2 ml d'eau bidistillée et on ajoute 100 mg de sérum albumine bovine dissoute dans 2 ml de PBS et 10 mg de carbodiimide soluble (1-éthyl-3-(3-diméthylamino-propyl)-carbodiimide, Pierce Chem., USA) dissous dans 1 ml de PBS. Le mélange est agité lentement à 4°C pendant 16 h à l'abri de la lumière puis la drogue couplée est séparée de la drogue libre par tamisage moléculaire sur une colonne de Biogel P-100® (Biorad Laboratoires, USA). Le premier pic élué contient 12,6 $\mu$g drogue/ml et est récupéré dans un volume de 46 ml. Ce qui donne un rendement de couplage de 16,9% et un rapport molaire drogue/protéine de 1,4. La solution est filtrée sur Millipore® 0,2 $\mu$m et conservée à 4°C.

Exemple 3

1. Préparation de la fétuine succinylée

On dissout 1 g de fétuine (Sigma-type III), dans laquelle 1 g de fétuine correspond à 0,33 mmoles d'amines libres, dans 10 $cm^3$ d'eau bidistillée. Le pH est ajusté à 7,5 par addition de soude 0,5 N. A la solution on ajoute 68 mg d'anhydride succinique (0,68 $\mu$mole) par petites fractions en maintenant le pH au voisinage de 7,5 par addition de sonde N. On ajoute à nouveau 68 mg d'anhydride succinique puis la solution est dialysée contre 3 fois 2 litres d'eau distillée à 4°C.

2. Couplage avec la L-alanyl-L-leucyl-L-alanyl-L-leucyl-daunorubicine

On dissout 9,6 mg (10,3 $\mu$moles) de chlorhydrate de L-alanyl-L-leucyl-L-alanyl-L-leucyl-daunorubicine dans 2,5 $cm^3$ d'eau puis on ajoute 2,5 $cm^3$ d'une solution de fétuine succinylée à 20 mg/$cm^3$ (1,04 $\mu$mole). On ajoute ensuite 3 mg (15,6 $\mu$moles) d'éthyl-1 (diéthylamino-3 propyl)-3 carbodiimide. Le mélange réactionnel est laissé au repos et à l'obscurité pendant 6 heures à 4°C. On ajoute alors 1,5 mg d'éthyl-1 (diéthylamino-3 propyl)-3 carbodiimide puis on abandonne pendant 15 heures à une température voisine de 20°C.

La solution est alors filtrée sur une colonne de Biogel P-100® pour séparer la drogue libre de la drogue couplée. On obtient un rendement de couplage de 83% et le rapport molaire drogue/protéine est voisin de 7.

Exemple 4

1. Préparation de la L-leucyl-L-alanyl-L-leucyl-doxorubicine

a. On agite pendant 24 heures un mélange de 120 mg de chlorhydrate de L-leucyl-doxorubicine* et 80 mg de N-trityl-L-alaninate de N-hydroxysuccinimide dans 4 $cm^3$ de diméthylformamide en présence de 0,024 $cm^3$ de triéthylamine. L'évolution de la réaction est suivie par chromatographie en couche mince. Lorsque la réaction est terminée le solvant est évaporé sous pression réduite. Le résidu obtenu est purifié par chromatographie sur colonne de 10 g de silica gel en éluant par des mélanges chloroformeméthanol contenant progressivement de 1 à 10% de méthanol.

L'éluat est récupéré en une série de fractions et celles contenant la N-trityl-L-alanyl-L-leucyl-doxorubicine sont rassemblées.

L'élimination du groupe protecteur de la fonction amine de la L-alanine est effectuée à température ambiante au moyen d'acide acétique 75%. Après 20 minutes la solution est refroidie à 0°C, neutralisée par du $NH_4OH$ concentré. La solution est filtrée. Le précipité est lavé à l'eau distillée et le filtrat extrait au chloroforme et séché sur du $Na_2SO_4$ anhydre. Après évaporation du solvant sous pression réduite, le chlorhydrate de N-L-ala-L-leu-doxorubicine est obtenu par addition d'un équivalent d'HCl 1 N.

b. La N-L-leucyl-L-alanyl-L-leucyl-doxorubicine est obtenue en suivant la méthode décrite an a, mais en partant de 120 mg du chlorhydrate de N-L-alanyl-L-leucyl-doxorubicine et de 85 mg de N-trityl-L-leucinate de N-hydroxysuccinimide.

2. Préparation de la N-succinyl-L-leucyl-L-alanyl-L-leucyl-doxorubicine

La succinylation de la N-L-leucyl-L-alanyl-L-leucyl-doxorubicine est effectuée tel que décrit dans l'exemple 2a. pour la L-ala-L-leu-L-ala-L-leu-daunorubicine.

3. Couplage de la N-succinyl-L-leucyl-L-alanyl-L-leucyl-doxorubicine à la sérum albumine bovine

Le couplage est effectué tel que décrit dans l'exemple 2b. pour la N-succinyl-L-alanyl-L-leucyl-L-alanyl-L-leucyl-daunorubicine.

Exemple 5

1. Préparation de l'asialofétuine succinylée

Une solution de 1 g de fétuine (Sigma Chem., Etats-Unis) dans 10 $cm^3$ de tampon acétate 0,2 M à pH 5,5 est passée, en circuit fermé, à travers une colonne de neuraminidase insolubilisée (Sigma Chem., Etats-Unis) maintenue à 45°C.

Après 48 heures, la solution d'asialofétuine est récupérée; le gel est lavé par du chlorure de sodium 2 M et l'acide sialique est éliminé par dialyse de la solution pendant 12 heures contre de l'eau distillée. L'asialofétuine est purifiée par passage sur une colonne de 125 $cm^3$ de diéthylaminoéthyl cellulose qui est éluée par du tampon Tris 0,01 M à pH 8. Des fractions de 150 gouttes sont recueillies à l'aide d'un collecteur de fraction Gilson. Les tubes contenant l'asialofétuine sont réunis et la solution est dialysée contre de l'eau puis lyophilisée.

2. Préparation de la N-L-leucyl-primaquine

a. On agite pendant 24 heures un mélange de 600 mg de primaquine (2,321 mmoles) et de 1,5 g de fluorénylméthoxycarbonyl-L-leucinate de N-hydroxysuccinimide (3,36 mmoles) dans 15 $cm^3$ de diméthylformamide. L'évolution de la réaction est suivie par chromatographie en couche mince. Lorsque la réaction est terminée le solvant est évaporé sous pression réduite. Le résidu obtenu est purifié par chromatographie sur silicagel en éluant par des mélanges chloroforme-méthanol contenant progressivement de 1 à 10% de méthanol et enfin par un mélange chloroforme-méthanol-ammoniaque concentrée (900—100—8 en volumes). L'éluat est récupéré en une aérie de fractions qui sont examinées en chromatographie de couche mince, par rapport à des étalons après détection dans l'ultraviolet.

L'élimination du groupement protecteur de la fonction amine de la L-leucine est effectuée au moyen de pipéridine. Le précipité obtenu est séparé par filtration et le filtrat est neutralisé à pH 7 par une solution d'acide chlorhydrique N. La L-leucyl-primaquine est extraite par du chloroforme (200 $cm^3$). La phase organique est séchée sur sulfate de sodium. Après filtration et concentration sous pression réduite (2666,44 Pa), on obtient 647 mg de L-leucyl-primaquine. A 647 mg de L leucyl-primaquine (1,74 mmoles), on ajoute 3,48 $cm^3$ d'acide chlorhydrique 1 N. On obtient ainsi une solution de dichlorhydrate de L-leucyl-primaquine.

La N-L-leucyl-primaquine peut également être préparée de la manière suivante.

---

*La préparation et les propriétés de la L-leucyl-doxorubicine ont été décrites dans le brevet belge n° 869.485.

b. A une solution de 7,68 g de primaquine (0,030 mole) dans 125 $cm^3$ de chloroforme sec refroidie à 0°C on ajoute, goutte à goutte, 4,65 g de N-carboxyanhydride de L-leucine (0,030 mole) dans 50 $cm^3$ d'éther sec. La réaction est complète en 30 minutes. Après évaporation du solvant, le produit est purifié par chromatographie. On obtient ainsi 6,7 g de L-leucyl-primaquine.

3. Préparation de la L-alanyl-L-leucyl-primaquine

On agite un mélange de 4 g de L-leucyl-primaquine (10,75 mmoles) et de 4,65 g de trityl-L-alaninate de N-hydroxysuccinimide dans 30 $cm^3$ de diméthylformamide. L'évolution de la réaction est suivie par chromatographie en couche mince. Après évaporation du solvant sous pression réduite (2666,44 Pa), le produit est purifié par chromatographie sur silice en éluant par des mélanges chloroforme-méthanol contenant progressivement de 1 à 10% de méthanol.

On obtient ainsi 5,21 g de N-trityl-L-alanyl-L-leucyl-primaquine que l'on traite par 50 $cm^3$ d'acide acétique à 75%. Le précipité de triphényl-carbinol est séparé par filtration, puis le filtrat est neutralisé à froid par addition d'ammoniaque concentrée (50 $cm^3$) jusqu'à pH 7. Après extraction du mélange réactionnel par le chloroforme, la phase organique est séchée sur sulfate de sodium. Après filtration et évaporation du solvant sous pression réduite (2666,44 Pa), on obtient 3,45 g de L-alanyl-L-leucyl-primaquine. A 3,45 g de L-alanyl-L-leucyl-primaquine (7,78 mmoles), on ajoute 15,58 $cm^3$ d'une solution d'acide chlorhydrique normal. On obtient ainsi une solution de dichlorhydrate de L-alanyl-L-leucyl-primaquine qui est lyophilisée.

4. Couplage de la N-L-alanyl-L-leucyl-primaquine à l'asialofétuine succinylée

On dissout 332 mg d'asialofétuine ($7.10^{-3}$ mmoles) dans 2,5 $cm^3$ de tampon phosphate (Phosphate Buffered Saline) et on ajoute 50 mg de dichlorhydrate de L-alanyl-L-leucyl-primaquine et 50 mg de éthyl-1 (diméthylamino-3 propyl)-3 carbodiimide. On laisse en contact pendant 15 heures à l'abri de la lumière et à une température voisine de 20°C. Le mélange réactionnel est filtré sur une colonne de 125 $cm^3$ de Biogel P-100®. On recueille des fractions de 150 gouttes.

L'asialofétuine-L-alanyl-L-leucyl-primaquine est recueillie dans les fractions 6 à 12.

Exemple 6

1. Préparation de la N-L-leucyl-L-alanyl-L-leucyl-primaquine.

La synthèse du dérivé tripeptidique de la primaquine est effectuée tel que décrit dans l'exemple 5 par. 3, à partir de N-L-alanyl-L-leucyl-primaquine obtenue tel que décrit dans l'exemple 5 par. 3 et du N-trityl-L-leucinate de N-hydroxysuccinimide.

2. Préparation de la N-L-alanyl-L-leucyl-L-alanyl-L-leucyl-primaquine.

Ce dérivé tétrapeptidique de la primaquine est préparé tel que décrit dans l'exemple 5 par. 3, à partir de N-L-leucyl-L-alanyl-L-leucyl-primaquine obtenue tel que décrit dans l'exemple 6, par. 1 et du N-trityl-L-alaninate de N-hydroxysuccinimide. Le dichlorhydrate de N-L-alanyl-L-leucyl-L-alanyl-L-leucyl-primaquine possède un Rf de 0,29 dans le système de C.C.M.: $CHCl_3$—MeOH—$NH_4OH$ 26% (900:100:9; v/v) et de 0,34 dans le système: $CHCl_3$—MeOH—$H_2O$ (120:20:1; v/v).

3. Couplage de la N-L-alanyl-L-leucyl-L-alanyl-L-leucyl-primaquine à l'asialofétuine succinylée

Le couplage du dérivé tétrapeptidique de la primaquine à l'asialofétuine succinylée est réalisé tel que décrit dans l'exemple 5, par. 4, pour le couplage à cette protéine de la N-L-alanyl-L-leucyl-primaquine.

La présente invention concerne également les compositions médicinales contenant les nouvelles formes pharmaceutiques selon l'invention seules ou en association avec un ou plusieurs diluants ou adjuvants compatibles. Ces compositions pourront être administrées par voie parentérale.

Les compositions selon l'invention pour administration parentérale peuvent être des solutions stériles aqueuses ou non aqueuses, des suspensions ou des émulsions. Comme solvant ou véhicule, on peut employer le propylène-glycol, un polyéthylène-glycol, des huiles végétales, en particulier l'huile d'olive, ou des esters organiques injectables, par exemple l'oléate d'éthyle. Ces compositions peuvent également comprendre des agents mouillants, émulsifiants et dispersants. La stérilisation peut se faire de plusieurs façons, par exemple à l'aide d'un filtre bactériologique, en incorporant à la composition des agents stérilisants ou par irradiation. Elles peuvent être également préparées sous forme de compositions solides stériles qui peuvent être dissoutes au moment de l'emploi dans de l'eau stérile ou tout autre milieu stérile injectable.

L'exemple suivant illustre une composition selon l'invention.

Exemple

On prépare selon la technique habituelle 100 $cm^3$ d'une solution stérile contenant une quantité de BSA-succinyl-L-ala-L-leu-L-ala-L-leu-daunorubicine correspondant à 100 mg de daunorubicine libre. Cette solution est administrée en perfusion lente.

**Revendications**

1. Forme pharmaceutique d'un médicament représentee schématiquement par:

Vecteur—Bras—Drogue

dans lequel:

— "drogue" représente un principe actif qui contient une fonction amine libre, choisi notamment parmi les anthracyclines antitumorales et les quinoléines antimalariques et antileishmaniales,
— "vecteur" est une macromolécule de nature protéinique endocytée sélectivement par les cellules cibles;

caractérisée en ce que:

— "bras" est une chaîne de nature peptidique représentée par l'enchaînement

X—L—Leu

dans lequel L-Leu représente un reste L-leucyl lié par sa fonction carboxylique à la fonction amine de la drogue et X représente 1, 2 ou 3 aminoacides, identiques ou différents, choisis parmi la L-alanine, la glycine ou la L-leucine, dont une fonction amine terminale est éventuellement succinylés et le rapport molaire drogue/vecteur est compris entre 1 et environ 7.

2. Forme pharmaceutique selon la revendication 1, caractérisée en ce que la drogue est choisie parmi la daunorubicine, la doxorubicine et la primaquine.

3. Forme pharmaceutique selon l'une des revendications 1 ou 2, caractérisée en ce que le bras contient l'enchaînement L-ala-L-leu, L-leu-L-ala-L-leu, L-ala-L-leu-L-ala-L-leu, $(L\text{-}ala)_3$-L-leu, gly-L-leu-gly-L-leu, L-ala-L-leu-gly-L-leu.

4. Forme pharmaceutique selon l'une des revendications 1, 2 ou 3, caractérisée en ce que le vecteur est choisi parmi le sérum albumine bovine, la fétuine, l'immunoglobuline, l'asialofétuine, ou les hormones peptidiques telles que la lactotropine ou l'hormone placentaire lactogène.

5. Procédé de préparation d'une forme pharmaceutique selon la revendication 1 caractérisé en ce que l'on fait réagir un produit de formule générale:

X—L—leu—Drogue

dans laquelle "Drogue" et X-L-leu sont définis comme dans la revendication 1, sur une protéine dans les conditions appropriées pour créer une liaison peptidique entre la fonction amine libre de l'aminoacide terminal du produit de formule générale X-L-leu-Drogue et les fonctions acides libres de la protéine, et isole le produit ainsi obtenu.

6. Procédé de préparation d'une forme pharmaceutique selon la revendication 1 caractérisé en ce que l'on fait réagir le produit de formule générale:

HO—succinyl—X—L—leu—drogue

dans laquelle "drogue" et X-L-leu sont définis comme dans la revendication 1, obtenu par succinylation du produit de formule générale

X—L—leu—drogue

sur une protéine dans les conditions appropriées pour créer une liaison peptidique entre la fonction carboxyle libre du succinyl terminal du produit de formule générale

HO—succinyl—X—L—leu—drogue

et les fonctions amines libres de la protéine, et isole le produit ainsi obtenu.

7. Procédé de préparation d'une forme pharmaceutique selon la revendication 1 caractérisé en ce que l'on fait réagir une protéine succinylée, obtenu par succinylation de la protéine vectrice, sur un produit de formule générale:

X—L—leu—drogue

dans laquelle X et "drogue" sont définis comme dans la revendication 1 dans les conditions appropriées pour créer une liaison peptidique entre les fonctions carboxyles libres de la protéine succinylée et la fonction amine libre du produit de formule générale:

X—L—Leu—drogue

et isole le produit obtenu.

8. Procédé de préparation d'une forme pharmaceutique selon l'une quelconque des revendications 5 à 7, caractérisé en ce que la réaction s'effectue dans un milieu tamponné convenable tel qu'un milieu au tampon phosphate (Phosphate Buffered Saline) en présence d'un agent de condensation tel qu'un carbodiimide comme l'éthyl-1 (diméthylamino-3 propyl)-3 carbodiimide à une température comprise entre 0 et 30°C.

9. Procédé suivant la revendication 7, caractérisé en ce qu'on opère en l'absence de lumière.

10. A titre de moyen pour la mise en oeuvre du procédé selon la revendication 6, le produit de formule générale:

X—L—leu—Drogue

dans laquelle "Drogue et X-L-leu sont tels que définis comme dans la revendication 1.

11. Produit suivant la revendication 10, caractérisé en ce qu'il est obtenu par condensation d'un aminoacide convenable ou d'un peptide sous forme acide dont les fonctions amines sont protégées, avec une drogue portant une fonction amine libre ou un dérivé de cette drogue portant une fonction amine libre en présence d'un agent de condensation selon les méthodes habituelles utilisées en chimie peptidique.

12. Composition médicinale caractérisée en ce qu'elle est constituée par la forme pharmaceutique selon la revendication 1, éventuellement en association avec un ou plusieurs diluants on adjuvants compatibles.

**Patentansprüche**

1. Pharmazeutische Form eines Arzneimittels, schematisch dargestellt durch:

Vektor—Arm—Arzneistoff,

worin:

"Arzneistoff" ein wirksames Prinzip mit einer freien Aminofunktion, das insbesondere aus Antitumor-Anthracyclinen und Anitmalaria- und Antileishman-Chinoleinen ausgewählt ist,
"Vektor" ein proteinartiges Makromolekül, das selektiv von Zielzellen einverleibt wird, darstellen,

dadurch gekennzeichnet, daß

"Arm" eine peptidartige Kette bedeutet, die durch die Verkettung

X—L—Leu

dargestellt ist, worin L-Leu einen L-Leucylrest, der durch seine Carboxylfunktion an die Aminofunktion des Arzneistoffs gebunden ist, und X 1, 2 oder 3 gleiche oder voneinander verschiedene Aminosäuren bedeutet, die aus L-Alanin, Glycin oder L-Leucin ausgewählt sind, deren eine endständige Aminofunktion gegebenenfalls succinyliert ist, und wobei das Mol-Verhältnis zwischen Arzneistoff und Vektor zwischen 1 und etwa 7 liegt.

2. Pharmazeutische Form nach Anspruch 1, dadurch gekennzeichnet, daß der Arzneistoff aus Daunorubicin, Doxorubicin und Primaquin ausgewählt ist.

3. Pharmazeutische Form nach einem der Ansprüche 1 oder 2, dadurch gekennzeichnet, daß der "Arm" die Verkettung L-Ala-L-Leu, L-Leu-L-Ala-L-Leu, L-Ala-L-Leu-L-Ala-L-Leu, $(L\text{-}Ala)_3$-L-Leu, Gly-L-Leu-Gly-L-Leu, L-Ala-L-Leu-Gly-L-Leu enthält.

4. Pharmazeutische Form nach einem der Ansprüche 1, 2 oder 3, dadurch gekennzeichnet, daß der "Vektor" aus Rinderserumalbumin, Fetuin, Immunoglobulin, Asialofetuin oder peptidartigen Hormonen, wie Lactotropin oder dem lactogenen Placentahormon ausgewählt ist.

5. Verfahren zur Herstellung einer neuen pharmazeutischen Form nach Anspruch 1, dadurch gekennzeichnet, daß man ein Produkt der allgemeinen Formel

X—L—Leu—Arzneistoff,

worin "Arzneistoff" und X-L-Leu wie in Anspruch 1 definiert sind, mit einem Protein unter geeigneten Bedingungen umsetzt, um eine Peptidbindung zwischen der freien Aminofunktion der endständigen Aminosäure des Produkts der allgemeinen Formel

X—L—Leu—Arzneistoff

und den freien Säurefunktionen des Proteins zu bilden, und das so erhaltene Produkt isoliert.

6. Verfahren zur Herstellung einer pharmazeutischen Form nach Anspruch 1, dadurch gekennzeichnet, daß man das Produkt der allgemeinen Formel

HO—Succinyl—X—L—Leu—Arzneistoff,

worin "Arzneistoff" und X-L-Leu wie in Anspruch 1 definiert sind und das durch Succinylierung des Produktes der allgemeinen Formel

X—L—Leu—Arzneistoff

erhalten worden ist, mit einem Protein unter geeigneten Bedingungen, um eine Peptidbindung zwischen der freien Carboxylfunktion des endständigen Succinyls des Produkts der allgemeinen Formel

HO—Succinyl—X—L—Leu—Arzneistoff

und den freien Aminofunktionen des Proteins zu bilden, umsetzt, und das so erhaltene Produkt isoliert.

7. Verfahren zur Herstellung einer pharmazeutischen Form nach Anspruch 1, dadurch gekennzeichnet, daß man ein succinyliertes Protein, das durch Succinylierung des "Vektor"-Proteins erhalten werden ist, mit einem Produkt der allgemeinen Formel

X—L—Leu—Arzneistoff

worin X und "Arzneistoff" wie in Anspruch 1 definiert sind, unter geeigneten Bedingungen umsetzt, um eine Peptidbindung zwischen den freien Carboxylfunktionen des succinylierten Proteins und der freien Aminofunktion des Produkts der allgemeinen Formel

X—L—Leu—Arzneistoff

zu bilden, und das erhaltene Produkt isoliert.

8. Verfahren zur Herstellung einer pharmazeutischen Form nach einem der Ansprüche 5 bis 7, dadurch gekennzeichnet, daß man die Umsetzung in einem geeigneten gepufferten Medium, zum Beispiel einem phosphatgepufferten Medium (phosphatgepufferter Kochsalzlösung) in Gegenwart eines Kondensationsmittels, zum Beispiel einem Carbodiimid, wie Ethyl-1-(Dimethylamino-3-propyl)-3-carbodiimid, bei einer Temperatur zwischen 0 und 30°C durchführt.

9. Verfahren nach Anspruch 7, dadurch gekennzeichnet, daß man in Abwesenheit von Licht arbeitet.

10. Als Mittel zur Durchführung des Verfahrens nach Anspruch 6, das Produkt der allgemeinen Formel

X—L—Leu—Arzneistoff,

worin "Arzneistoff" und X-L-Leu wie in Anspruch 1 definiert sind.

11. Produkt nach Anspruch 10, dadurch gekennzeichnet, daß es durch Kondensation einer geeigneten Aminosäure oder eines geeigneten Peptide in Säureform, deren Aminofunktionen geschützt sind, mit einem Arzneistoff, der eine freie Aminofunktion trägt, oder mit einem Derivat dieses Arzneistoffs, der eine freie Aminofunktion trägt, in Gegenwart eines Kondensationsmittels nach den in der Peptidchemie üblicherweise angewendeten Verfahren erhalten worden ist.

12. Medizinisches Präparat, dadurch gekennzeichnet, daß es aus der pharmazeutischen Form nach Anspruch 1, gegebenenfalls zusammen mit einem oder mehreren damit verträglichen Verdünnungsmitteln oder Adjuvantien zusammengesetzt ist.

## Claims

1. Pharmaceutical form of a medicament which is represented schematically by:

Carrier—Spacer arm—Drug

in which:

— "drug" represents an active principle containing a free amino function, selected among others from antitumoral anthracyclines, antimalarial and antileishmanial quinoleines,
— "carrier" is a macromolecule of proteinic nature which is selectively endocytosed by the target cells characterized in that
— "arm" is a chain of peptidic nature which is represented by the sequence

X—L—Leu

in which L-Leu represents a L-leucyl moiety bound through its carboxylic function to the amino function of the drug and X represents 1, 2 or 3 aminoacids which may be identical or different and are selected from L-alanine, glycine and L-leucine, and having a possibly succinylated terminal amino function, and the molar ratio drug/carrier is comprised between a and about 7.

2. Pharmaceutical form according to claim 1, characterized in that the drug is selected from daunorubicine, doxorubicine and primaquine.

3. Pharmaceutical form according to any of claims 1 or 2, characterized in that the arm contains the sequence L-ala-L-leu, L-leu-L-ala-L-leu, L-ala-L-leu-L-ala-L-leu, $(L\text{-}ala)_3$-L-leu, gly-L-leu-gly-L-leu, L-ala-L-leu-gly-L-leu.

4. Pharmaceutical form according to any of claims 1, 2 or 3, characterized in that the carrier is selected from bovine serum albumine, fetuine, immunoglobuline, asialofetuine or peptidic hormones such as lactotropine or lactogenous placentary hormone.

5. Process for preparing a pharmaceutical form according to claim 1, characterized in that a product of the general formula

X—L—leu—Drug

in which "Drug" and X-L-leu are defined as in claim 1, is reacted with a protein under appropriate conditions for creating a peptidic bond between the free amino function of the terminal aminoacid of the product of general formula X-L-leu-Drug and the free acid functions of the protein, and the product thus obtained is isolated.

6. Process for preparing a pharmaceutical form according to claim 1, characterized in that the product of general formula

HO—succinyl—X—L—leu—drug

in which "drug" and X-L-leu are defined as in claim 1, obtained by succinylation of the product of general formula

X—L—leu—drug

is reacted with a protein under appropriate conditions for creating a peptidic bond between the free carboxylic function of the terminal succinyl group of the product of the general formula

HO—succinyl—X—L—leu—drug

and the free amino functions of the protein and the product thus obtained is isolated.

7. Process for preparing a pharmaceutical form according to claim 1, characterized in that a succinylated protein obtained by succinylation of the carrier protein is reacted with a product of general formula

X—L—leu—drug

in which X and "drug" are defined as in claim 1 under appropriate conditions for creating a peptidic bond between the free carboxylic functions of the succinylated protein and the free amino function of the product of general formula

X—L—leu—drug

and the product obtained is isolated.

8. Process for preparing a pharmaceutical form according to any of claims 5 to 7, characterized in that the reaction is effected in a suitable medium of phosphate buffer (Phosphate Buffered Saline) in the presence of a condensation agent such as a carbodiimide as 1-ethyl-3-(3-dimethylaminopropyl)-carbodiimide at a temperature between 0 and 30°C.

9. Process according to claim 7, characterized in that the operation is effected in the absence of light.

10. As a means for applying the process according to claim 6, the product of general formula

X—L—leu—Drug

in which "Drug" and X-L-Leu are as defined in claim 1.

11. Product according to claim 10, characterized in that it is obtained by condensation of an appropriate aminoacid or a peptide in acid form the amino functions of which are protected, with a drug carrying a free amino function or a derivative of said drug carrying a free amino function in the presence of a condensation agent according to the usual methods used in peptidic chemistry.

12. Medicinal composition, characterized in that it consists of the pharmaceutical form according to claim 1, possibly in association with one or more compatible diluents or auxiliary agents.